# EUROPEAN PATENT APPLICATION

(11) **EP 3 446 741 A1**
(43) Date of publication of application: **27.02.2019**
(21) Application number: 18190704.9
(22) Date of filing: 24.08.2018
(51) Int. Cl.: A61M 25/09

(54) **CORE WIRE ASSEMBLY FOR GUIDEWIRE**

(30) Priority: 25.08.2017 US 201715686796
(71) Applicant: Acclarent, Inc., Irvine, CA 92618 (US)
(72) Inventor: MATLOCK, George L., Irvine, CA California 92618 (US)
(74) Representative: Small, Gary James

(57) **Abstract**

An apparatus and method of manufacture includes a helical wire coil and a core wire assembly. The core wire assembly includes a core wire and a sleeve. The core wire extends through the helical wire coil and is formed from a first material that is non-extensible. The sleeve has a longitudinal bore. The core wire is disposed within the longitudinal bore of the sleeve. The core wire is secured within the sleeve. The sleeve is formed from a second material. The second material of the sleeve is different than the first material of the core wire. The sleeve is attached to the distal wire coil such that core wire inhibits longitudinal elongation of the proximal and distal wire coils along the longitudinal coil axis.

## Description

### BACKGROUND

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guidewire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus. A system that may be used to perform such procedures may be provided in accordance with the teachings of U.S. Pub. No. 2011/0004057, entitled "Systems and Methods for Transnasal Dilation of Passageways in the Ear, Nose or Throat," published January 6, 2011, the disclosure of which is incorporated by reference herein. An example of such a system is the Relieva® Spin Balloon Sinuplasty™ System by Acclarent, Inc. of Irvine, California.

A variable direction view endoscope may be used with such a system to provide visualization within the anatomical passageway (e.g., the ear, nose, throat, paranasal sinuses, etc.) to position the balloon at desired locations. A variable direction view endoscope may enable viewing along a variety of transverse viewing angles without having to flex the shaft of the endoscope within the anatomical passageway. Such an endoscope that may be provided in accordance with the teachings of U.S. Pub. No. 2010/0030031, entitled "Swing Prism Endoscope," published February 4, 2010, the disclosure of which is incorporated by reference herein. An example of such an endoscope is the Acclarent Cyclops™ Multi-Angle Endoscope by Acclarent, Inc. of Irvine, California.

While a variable direction view endoscope may be used to provide visualization within the anatomical passageway, it may also be desirable to provide additional visual confirmation of the proper positioning of the balloon before inflating the balloon. This may be done using an illuminating guidewire. Such a guidewire may be positioned within the target area and then illuminated, with light projecting from the distal end of the guidewire. This light may illuminate the adjacent tissue (e.g., hypodermis, subdermis, etc.) and thus be visible to the naked eye from outside the patient through transcutaneous illumination. For instance, when the distal end is positioned in the maxillary sinus, the light may be visible through the patient's cheek. Using such external visualization to confirm the position of the guidewire, the balloon may then be advanced distally along the guidewire into position at the dilation site. Such an illuminating guidewire may be provided in accordance with the teachings of U.S. Pat. No. 9,155,492, entitled "Sinus Illumination Lightwire Device," issued October 13, 2015, the disclosure of which is incorporated by reference herein. An example of such an illuminating guidewire is the Relieva Luma Sentry™ Sinus Illumination System by Acclarent, Inc. of Irvine, California.

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.) so as to superimpose the current location of the instrument on the preoperatively obtained images. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit electromagnetic fields and/or are responsive to externally generated electromagnetic fields) mounted thereon are used to perform the procedure while the sensors send data to the computer indicating the current position of each surgical instrument. The computer correlates the data it receives from the instrument-mounted sensors with the digital map that was created from the preoperative tomographic scan. The tomographic scan images are displayed on a video monitor along with an indicator (e.g., cross hairs or an illuminated dot, etc.) showing the real time position of each surgical instrument relative to the anatomical structures shown in the scan images. In this manner, the surgeon is able to know the precise position of each sensor-equipped instrument by viewing the video monitor even if the surgeon is unable to directly visualize the instrument itself at its current location within the body.

Examples of electromagnetic IGS systems that may be used in ENT and sinus surgery include the InstaTrak ENT™ systems available from GE Medical Systems, Salt Lake City, Utah. Other examples of electromagnetic image guidance systems that may be modified for use in accordance with the present disclosure include but are not limited to the CARTO® 3 System by Biosense-Webster, Inc., of Irvine, California; systems available from Surgical Navigation Technologies,F Inc., of Louisville, Colorado; and systems available from Calypso Medical Technologies, Inc., of Seattle, Washington.

When applied to functional endoscopic sinus surgery (FESS), balloon sinuplasty, and/or other ENT procedures, the use of image guidance systems allows the surgeon to achieve more precise movement and positioning of the surgical instruments than can be achieved by viewing through an endoscope alone. This is so because a typical endoscopic image is a spatially limited, 2 dimensional, line-of-sight view. The use of image guidance systems provides a real time, 3-dimensional view of all of the anatomy surrounding the operative field, not just that which is actually visible in the spatially limited, 2 dimensional, direct line-of-sight endoscopic view. As a result, image guidance systems may be particularly useful during performance of FESS, balloon sinuplasty, and/or other ENT procedures where a section and/or irrigation source may be desirable, especially in cases where normal anatomical landmarks are not present or are difficult to visualize endoscopically.

While several systems and methods have been made and used in ENT procedures, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1A depicts a perspective view of an exemplary dilation instrument assembly, with an exemplary guidewire in a proximal position, and with a dilation catheter in a proximal position;
FIG. 1B depicts a perspective view of the dilation instrument assembly of FIG. 1A, with the guidewire in a distal position, and with the dilation catheter in the proximal position;
FIG. 1C depicts a perspective view of the dilation instrument assembly of FIG. 1A, with the guidewire in a distal position, with the dilation catheter in a distal position, and with a dilator of the dilation catheter in a non-dilated state;
FIG. 1D depicts a perspective view of the dilation instrument assembly of FIG. 1A, with the guidewire in a distal position, with the dilation catheter in the distal position, and with a dilator of the dilation catheter in a dilated state;
FIG. 2 depicts a schematic view of an exemplary image guided surgery (IGS) navigation system for use with the dilation instrument assembly of FIG. 1A;
FIG. 3 depicts a perspective view of the head of a patient, with components of the navigation system of FIG. 2;
FIG. 4 depicts a side elevational view of an exemplary illuminating guidewire for use in the dilation instrument assembly of FIG. 1A;
FIG. 5 depicts an enlarged side elevational view of the illuminating guidewire of FIG. 4;
FIG. 6 depicts an enlarged side cross-sectional view of the illuminating guidewire of FIG. 4 taken along a centerline thereof;
FIG. 7 depicts a side elevational view of a distal portion of an exemplary alternative guidewire for use in the dilation instrument assembly of FIG. 1A;
FIG. 8 depicts an enlarged side elevational view of the distal portion of the guidewire of FIG. 7 indicated by the "FIG. 8" broken line circle of FIG. 7;
FIG. 9 depicts an enlarged, partially exploded cross-sectional view of the guidewire of FIG. 7, taken along a centerline thereof;
FIG. 10 depicts an enlarged cross-sectional view of the guidewire of FIG. 7 taken along a centerline thereof; and
FIG. 11 depicts a cross-sectional side view of a bent region of the distal portion of the guidewire of FIG. 7, taken along a centerline thereof, indicated by the "FIG. 11" broken line circle of FIG. 7.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Overview of Exemplary Dilation Catheter System

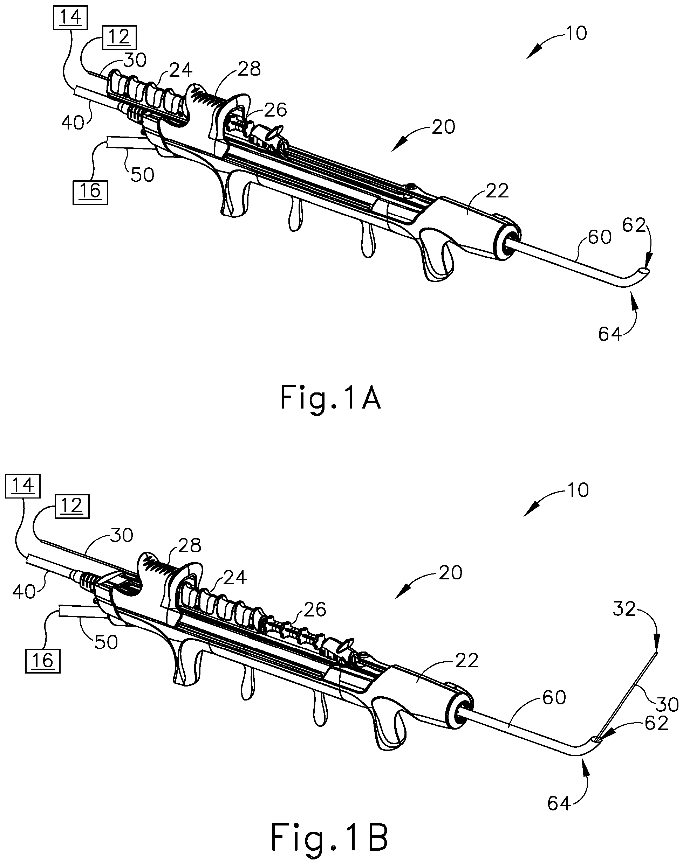

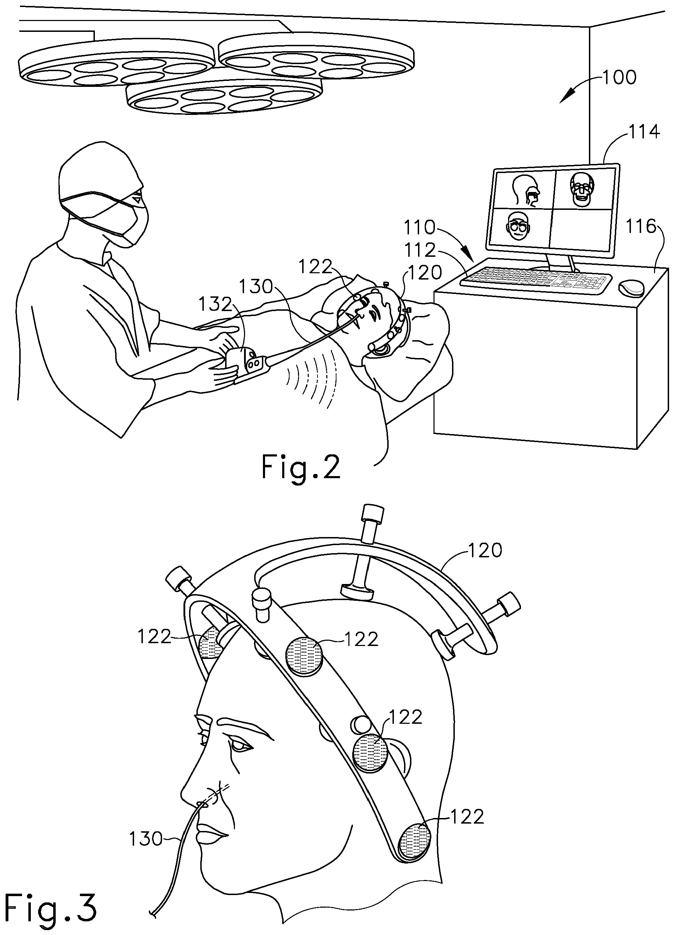

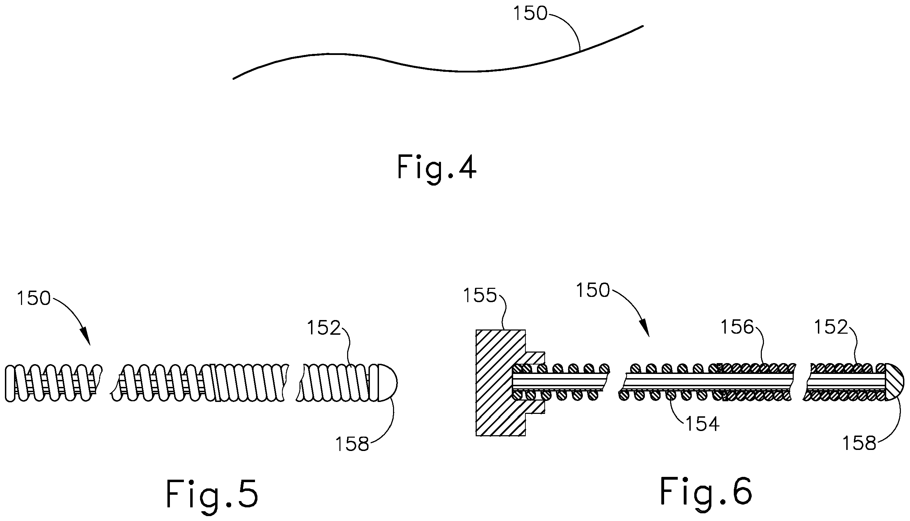

FIGS. 1A-1D shows a first exemplary dilation instrument assembly (10) that may be used to dilate the ostium of a paranasal sinus; to dilate some other passageway associated with drainage of a paranasal sinus; to dilate a Eustachian tube; or to dilate some other anatomical passageway (e.g., within the ear, nose, or throat, etc.). Dilation instrument assembly (10) of this example comprises a guidewire power source (12), an inflation source (14), an irrigation fluid source (16), and a dilation instrument (20). In some versions, guidewire power source (12) is part of an IGS system as described below with respect to FIGS. 2-3. In some other versions, guidewire power source (12) comprises a source of light as described below with respect to FIGS. 4-6. In the present example shown in FIGS. 1A-1D, inflation source (14) comprises a source of saline. However, it should be understood that any other suitable source of fluid (liquid or otherwise) may be used. Also in the present example, irrigation fluid source (16) comprises a source of saline. Again, though, any other suitable source of fluid may be used. It should also be understood that flush fluid source (16) may be omitted in some versions.

Dilation instrument (20) of the present example comprise a handle body (22) with a guidewire slider (24), a guidewire spinner (26), and a dilation catheter slider (28). Handle body (22) is sized and configured to be gripped by a single hand of a human operator. Sliders (24, 28) and spinner (26) are also positioned and configured to be manipulated by the same hand that grasps handle body (22). It should therefore be understood that dilation instrument (20) may be fully operated by a single hand of a human operator.

### A. Exemplary Guide Catheter

A guide catheter (60) extends distally from handle body (22). Guide catheter (60) includes an open distal end (62) and a bend (64) formed proximal to open distal end (62). In the present example, dilation instrument (20) is configured to removably receive several different kinds of guide catheters (60), each guide catheter (60) having a different angle formed by bend (64). These different angles may facilitate access to different anatomical structures. Various examples of angles and associated anatomical structures are described in one or more of the references cited herein; while further examples will be apparent to those of ordinary skill in the art in view of the teachings herein. Guide catheter (60) of the present example is formed of a rigid material (e.g., rigid metal and/or rigid plastic, etc.), such that guide catheter (60) maintains a consistent configuration of bend (64) during use of dilation instrument (20). In some versions, dilation instrument (20), is further configured to enable rotation of guide catheter (60), relative to handle body (22), about the longitudinal axis of the straight proximal portion of guide catheter (60), thereby further promoting access to various anatomical structures.

### B. Exemplary Guidewire

Dilation instrument (30) further comprises an exemplary guidewire (30), which is coaxially disposed in guide catheter (60). Guidewire slider (24) is secured to guidewire (30) such that translation of guidewire slider (24) relative to handle body (22) provides corresponding translation of guidewire (30) relative to handle body (22). In particular, translation of guidewire slider (24) from a proximal position (FIG. 1A) to a distal position (FIG. 1B) causes corresponding translation of guidewire (30) from a proximal position (FIG. 1A) to a distal position (FIG. 1B). When guidewire (30) is in a distal position, a distal portion of guidewire (30) protrudes distally from open distal end (62) of guide catheter (60). Guidewire spinner (26) is operable to rotate guidewire (30) about the longitudinal axis of guidewire (30). Guidewire spinner (26) is coupled with guidewire slider (24) such that guidewire spinner (26) translates longitudinally with guidewire slider (24).

In some versions, guidewire (30) includes a preformed bend formed just proximal to a distal end (32) of guidewire (30). In such versions, the preformed bend and the rotatability provided via guidewire spinner (26) may facilitate alignment and insertion of distal end (32) into a sinus ostium, Eustachian tube, or other passageway to be dilated. Also in some versions, guidewire (30) includes at least one optical fiber extending to a lens or other optically transmissive feature in distal end (32), such as illuminating guidewire (150) (*see* FIGS. 4-6) discussed below. Optical fiber may be in optical communication with guidewire power source (12), such that light may be communicated from guidewire power source (12) to distal end (32). In such versions, guidewire (30) may provide transillumination through a patient's skin in order to provide visual feedback to the operator indicating that distal end (32) has reached a targeted anatomical structure.

By way of example only, guidewire (30) may be configured in accordance with at least some of the teachings of U.S. Pat. No. 9,155,492, the disclosure of which is incorporated by reference herein. In some versions, guidewire (30) is configured similar to the Relieva Luma Sentry™ Sinus Illumination System by Acclarent, Inc. of Irvine, California. In addition to, or as an alternative to, including one or more optical fibers, guidewire (30) may include a sensor and at least one wire that enables guidewire (30) to provide compatibility with an IGS system as described in greater detail below. Other features and operabilities that may be incorporated into guidewire (30) will be apparent to those of ordinary skill in the art in view of the teachings herein.

### C. Exemplary Dilation Catheter

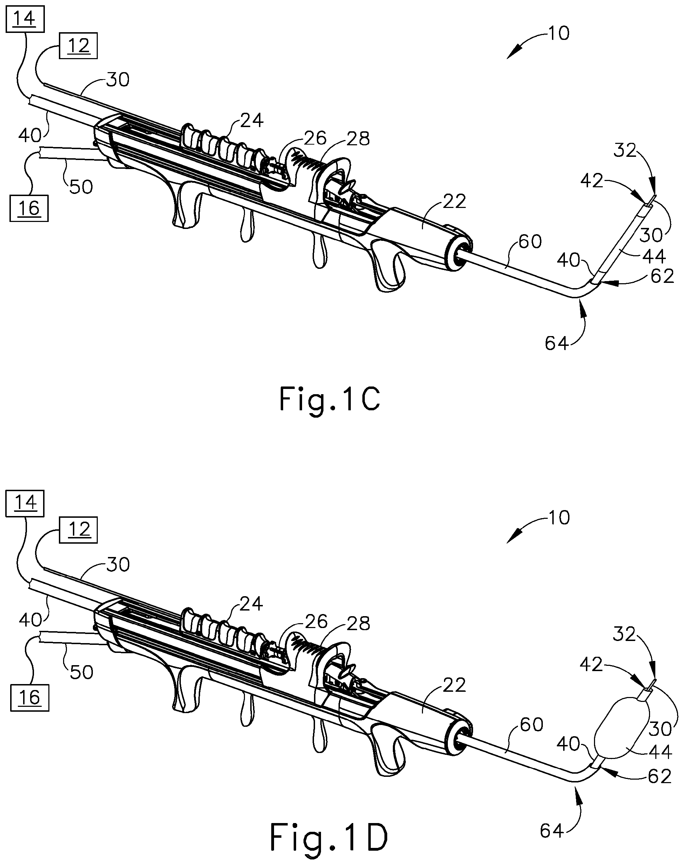

Dilation instrument (30) further comprises a dilation catheter (40), which is coaxially disposed in guide catheter (60). Dilation catheter slider (28) is secured to dilation catheter (40) such that translation of dilation catheter slider (28) relative to handle body (22) provides corresponding translation of dilation catheter (40) relative to handle body (22). In particular, translation of dilation catheter slider (28) from a proximal position (FIG. 1B) to a distal position (FIG. 1C) causes corresponding translation of dilation catheter (40) from a proximal position (FIG. 1B) to a distal position (FIG. 1C). When dilation catheter (40) is in a distal position, a distal portion of dilation catheter (40) protrudes distally from open distal end (62) of guide catheter (60). As can also be seen in FIG. 1C, a distal portion of guidewire (30) protrudes distally from the open distal end of dilation catheter (40) when guidewire (30) and dilation catheter are both in distal positions.

Dilation catheter (40) of the present example comprises a non-extensible balloon (44) located just proximal to an open distal end (42) of dilation catheter (40). Balloon (44) is in fluid communication with inflation source (14). Inflation source (14) is configured to communicate fluid (e.g., saline, etc.) to and from balloon (44) to thereby transition balloon (44) between a non-inflated state and an inflated state. FIG. 1C shows balloon (44) in a non-inflated state. FIG. 1D shows balloon (44) in an inflated state. In some versions, inflation source (14) comprises a manually actuated source of pressurized fluid. In some such versions, the manually actuated source of pressurized fluid is configured and operable in accordance with at least some of the teachings of U.S. Pub. No. 2014/0074141, entitled "Inflator for Dilation of Anatomical Passageway," published March 13, 2014, the disclosure of which is incorporated by reference herein. Other suitable configurations that may be used to provide a source of pressurized fluid will be apparent to those of ordinary skill in the art in view of the teachings herein.

While not shown, it should be understood that dilation catheter (40) may include at least two separate lumens that are in fluid isolation relative to each other. One lumen may provide a path for fluid communication between balloon (44) and inflation source (14). The other lumen may provide a path to slidably receive guidewire (30).

While dilation catheter (40) of the present example is configured to transition between a non-dilated state and a dilated state based on the communication of fluid to and from balloon (44), it should be understood that dilation catheter (40) may include various other kinds of structures to serve as a dilator. By way of example only, balloon (44) may be replaced with a mechanical dilator in some other versions. Dilation catheter (40) may be constructed and operable in accordance with any of the various references cited herein. In some versions, dilator catheter (40) is configured and operable similar to the Relieva Ultirra™ Sinus Balloon Catheter by Acclarent, Inc. of Irvine, California. In some other versions, dilator catheter (40) is configured and operable similar to the Relieva Solo Pro™ Sinus Balloon Catheter by Acclarent, Inc. of Irvine, California. Other suitable variations of dilation catheter (40) will be apparent to those of ordinary skill in the art in view of the teachings herein.

### D. Exemplary Irrigation Features

In some instances, it may be desirable to irrigate an anatomical site. For instance, it may be desirable to irrigate a paranasal sinus and nasal cavity after dilation catheter (40) has been used to dilate an ostium or other drainage passageway associated with the paranasal sinus. Such irrigation may be performed to flush out blood, etc. that may be present after the dilation procedure. In some such cases, guide catheter (60) may be allowed to remain in the patient while guidewire (30) and dilation catheter (40) are removed. A dedicated irrigation catheter (not shown) may then be inserted into guide catheter (60) and coupled with irrigation fluid source (16) via tube (50), to enable irrigation of the anatomical site in the patient. An example of an irrigation catheter that may be fed through guide catheter (60) to reach the irrigation site after removal of dilation catheter (60) is the Relieva Vortex® Sinus Irrigation Catheter by Acclarent, Inc. of Irvine, California. Another example of an irrigation catheter that may be fed through guide catheter (60) to reach the irrigation site after removal of dilation catheter (40) is the Relieva Ultirra® Sinus Irrigation Catheter by Acclarent, Inc. of Irvine, California.

In some other versions, dilation catheter (40) includes an additional irrigation lumen and an associated set of irrigation ports near distal end (42), such that dilation catheter (40) may be coupled with irrigation fluid source (16) via tube (50). Thus, a separate, dedicated irrigation catheter is not necessarily required in order to provide irrigation.

By way of example only, irrigation may be carried out in accordance with at least some of the teachings of U.S. Pat. No. 7,630,676, entitled "Methods, Devices and Systems for Treatment and/or Diagnosis of Disorders of the Ear, Nose and Throat," issued December 8, 2009, the disclosure of which is incorporated by reference herein. Of course, irrigation may be provided in the absence of a dilation procedure; and a dilation procedure may be completed without also including irrigation. It should therefore be understood that dilation fluid source (16) and tube (50) are merely optional.

### E. Exemplary Variations

In the present example, guidewire (30) is coaxially disposed within dilation catheter (40), which is coaxially disposed within guide catheter (60). In some other versions, guide catheter (60) is omitted from dilation instrument (20). In some such versions, a malleable guide member is used to guide guidewire (30) and dilation catheter (40). In some such versions, guidewire (30) is omitted and dilation catheter (40) is slidably disposed about the exterior of the internal malleable guide member. In some other versions, guidewire (30) is slidably disposed about the exterior of the internal malleable guide member; and dilation catheter (40) is slidably disposed about the exterior of guidewire (30). In still other versions, guidewire (30) is slidably disposed within the interior of the malleable guide member; and dilation catheter (40) is slidably disposed about the exterior of the malleable guide member.

By way of example only, versions of dilation instrument (20) that include a malleable guide member may be constructed and operable in accordance with at least some of the teachings of U.S. Pub. No. 2016/0310714, entitled "Balloon Dilation System with Malleable Internal Guide," published October 27, 2016, the disclosure of which is incorporated by reference herein. As another merely illustrative example, versions of dilation instrument (20) that include a malleable guide member may be constructed and operable in accordance with at least some of the teachings of U.S. Pat. App. No. 14/928,260, entitled "Apparatus for Bending Malleable Guide of Surgical Instrument," filed October 30, 2015, the disclosure of which is incorporated by reference herein; and/or U.S. Pub. No. 2012/0071857, entitled "Methods and Apparatus for Treating Disorders of the Sinuses," published March 22, 2012, the disclosure of which is incorporated by reference herein.

It should be understood that the variations of dilation instrument (20) described below in the context of an IGS system may be incorporated into versions of dilation instrument (20) having a malleable guide just like the variations of dilation instrument (20) described below in the context of an IGS system may be incorporated into versions of dilation instrument (20) having a rigid guide catheter (60).

Various examples below describe the use of an IGS system to provide navigation of instruments within a patient. In particular, various examples below describe how dilation instrument assembly (10) may be modified to incorporate IGS system features. However, it should also be understood that dilation instrument assembly (10) may be used in conjunction with conventional image guidance instruments, in addition to being used with IGS system components. For instance, dilation instrument assembly (10) may be used in conjunction with an endoscope, at least to provide initial positioning of guide catheter (60) in a patient. By way of example only, such an endoscope may be configured in accordance with at least some of the teachings of U.S. Pub. No. 2010/0030031, the disclosure of which is incorporated by reference herein. Other suitable kinds of endoscopes that may be used with the various versions of dilation instrument assembly (10) described herein will be apparent to those of ordinary skill in the art.

### II. Exemplary Guidance of Dilation Catheter System

### A. Image Guided Surgery Navigation System

FIG. 2 shows an exemplary IGS navigation system (100) whereby an ENT procedure may be performed using IGS. In some instances, IGS navigation system (100) is used during a procedure where dilation instrument assembly (10) that may be used to dilate the ostium of a paranasal sinus; or to dilate some other anatomical passageway (e.g., within the ear, nose, or throat, etc.). However, it should be understood that IGS navigation system (100) may be readily used in various other kinds of procedures.

In addition to or in lieu of having the components and operability described herein, IGS navigation system (100) may be constructed and operable in accordance with at least some of the teachings of U.S. Pat. No. 8,702,626, entitled "Guidewires for Performing Image Guided Procedures," issued April 22, 2014, the disclosure of which is incorporated by reference herein; U.S. Pat. No. 8,320,711, entitled "Anatomical Modeling from a 3-D Image and a Surface Mapping," issued November 27, 2012, the disclosure of which is incorporated by reference herein; U.S. Pat. No. 8,190,389, entitled "Adapter for Attaching Electromagnetic Image Guidance Components to a Medical Device," issued May 29, 2012, the disclosure of which is incorporated by reference herein; U.S. Pat. No. 8,123,722, entitled "Devices, Systems and Methods for Treating Disorders of the Ear, Nose and Throat," issued February 28, 2012, the disclosure of which is incorporated by reference herein; and U.S. Pat. No. 7,720,521, entitled "Methods and Devices for Performing Procedures within the Ear, Nose, Throat and Paranasal Sinuses," issued May 18, 2010, the disclosure of which is incorporated by reference herein.

Similarly, in addition to or in lieu of having the components and operability described herein, IGS navigation system (100) may be constructed and operable in accordance with at least some of the teachings of U.S. Pat. Pub. No. 2014/0364725, entitled "Systems and Methods for Performing Image Guided Procedures within the Ear, Nose, Throat and Paranasal Sinuses," published December 11, 2014, the disclosure of which is incorporated by reference herein; U.S. Pat. Pub. No. 2014/0200444, entitled "Guidewires for Performing Image Guided Procedures," published July 17, 2014, the disclosure of which is incorporated by reference herein; U.S. Pat. No. 9,198,736, entitled "Adapter for Attaching Electromagnetic Image Guidance Components to a Medical Device," issued December 1, 2015, the disclosure of which is incorporated by reference herein; U.S. Pat. Pub. No. 2011/0060214, entitled "Systems and Methods for Performing Image Guided Procedures within the Ear, Nose, Throat and Paranasal Sinuses," published March 10, 2011, the disclosure of which is incorporated by reference herein; U.S. Pat. No. 9,167,961, entitled "Methods and Apparatus for Treating Disorders of the Ear Nose and Throat," issued October 27, 2015, the disclosure of which is incorporated by reference herein; and U.S. Pat. Pub. No. 2007/0208252, entitled "Systems and Methods for Performing Image Guided Procedures within the Ear, Nose, Throat and Paranasal Sinuses," published September 6, 2007, the disclosure of which is incorporated by reference herein.

IGS navigation system (100) of the present example comprises a set of magnetic field generators (122). Before a surgical procedure begins, field generators (122) are fixed to the head of the patient. As best seen in FIG. 3, field generators (122) are incorporated into a frame (120), which is clamped to the head of the patient. While field generators (122) are secured to the head of the patient in this example, it should be understood that field generators (122) may instead be positioned at various other suitable locations and on various other suitable structures. By way of example only, field generators (122) may be mounted on an independent structure that is fixed to a table or chair on which the patient is positioned, on a floor-mounted stand that has been locked in position relative to the head of the patient, and/or at any other suitable location(s) and/or on any other suitable structure(s).

Field generators (122) are operable to generate an electromagnetic field around the head of the patient. In particular, field generators (122) are operated so as to transmit alternating magnetic fields of different frequencies into a region in proximity to frame (120). Field generators (122) thereby enable tracking of the position of a navigation guidewire (130) that is inserted into a nasal sinus of the patient and in other locations within the patient's head. Various suitable components that may be used to form and drive field generators (122) will be apparent to those of ordinary skill in the art in view of the teachings herein.

Navigation guidewire (130) may be used as a substitute for guidewire (30) described above, and may include a sensor (not shown) that is responsive to movement within the fields generated by field generators (122). In particular, with respect to FIG. 2, signals generated by the sensor of navigation guidewire (130) may be processed by a processor (110) to determine the three-dimensional location of navigation guidewire (130) within the patient. Various suitable forms that the sensor may take will be apparent to those of ordinary skill in the art in view of the teachings herein, particularly in view of several of the references that are cited herein in the context of IGS navigation system (100). It should be understood that, when used as a substitute for guidewire (30) in dilation instrument assembly (10), navigation guidewire (130) may facilitate navigation of instrumentation of dilation instrument assembly (10) within the patient during performance of a procedure to dilate the ostium of a paranasal sinus; or to dilate some other anatomical passageway (e.g., within the ear, nose, or throat, etc.). It should also be understood that other components of dilation instrument assembly (10) may incorporate a sensor like the sensor of navigation guidewire (130).

IGS navigation system (100) of the present example further comprises a processor (110), which controls field generators (122) and other elements of IGS navigation system (100). Processor (110) comprises a processing unit communicating with one or more memories. Processor (110) of the present example is mounted in a console (116), which comprises operating controls (112) that include a keypad and/or a pointing device such as a mouse or trackball. A physician uses operating controls (112) to interact with processor (110) while performing the surgical procedure.

Console (116) also connects to other elements of system (100). For instance, as shown in FIG. 2 a coupling unit (132) is secured to the proximal end of navigation guidewire (130). Coupling unit (132) of this example is configured to provide wireless communication of data and other signals between console (116) and navigation guidewire (130). In some versions, coupling unit (132) simply communicates data or other signals from navigation guidewire (130) to console (116) uni-directionally, without also communicating data or other signals from console (116). In some other versions, coupling unit (132) provides bidirectional communication of data or other signals between navigation guidewire (130) to console (116). While coupling unit (132) of the present example couples with console (116) wirelessly, some other versions may provide wired coupling between coupling unit (132) and console (116). Various other suitable features and functionality that may be incorporated into coupling unit (132) will be apparent to those of ordinary skill in the art in view of the teachings herein.

Processor (110) uses software stored in a memory of processor (110) to calibrate and operate system (100). Such operation includes driving field generators (122), processing data from navigational guidewire (130), processing data from operating controls (112), and a driving display screen (114). The software may be downloaded to processor (110) in electronic form, over a network, for example, or it may, alternatively or additionally, be provided and/or stored on non-transitory tangible media, such as magnetic, optical, or electronic memory.

Processor (110) is further operable to provide video in real time via display screen (114), showing the position of the distal end of navigational guidewire (130) in relation to a video camera image of the patient's head, a CT scan image of the patient's head, and/or a computer generated three-dimensional model of the anatomy within and adjacent to the patient's nasal cavity. Display screen (114) may display such images simultaneously and/or superimposed on each other. Moreover, display screen (114) may display such images during the surgical procedure. Such displayed images may also include graphical representations of instruments that are inserted in the patient's head, such as navigational guidewire (130), such that the operator may view the virtual rendering of the instrument at its actual location in real time. Such graphical representations may actually look like the instrument or may be a much simpler representation such as a dot, crosshairs, etc. By way of example only, display screen (114) may provide images in accordance with at least some of the teachings of U.S. Pub. No. 2016/0008083, entitled "Guidewire Navigation for Sinuplasty," published January 14, 2016, the disclosure of which is incorporated by reference herein. In the event that the operator is also using an endoscope, the endoscopic image may also be provided on display screen (114). The images provided through display screen (114) may help guide the operator in maneuvering and otherwise manipulating instruments within the patient's head.

In the present example, navigational guidewire (130) includes one or more coils at the distal end of navigational guidewire (130). Such a coil serves as a sensor as referred to above. When such a coil is positioned within an electromagnetic field generated by field generators (122), movement of the coil within that magnetic field may generate electrical current in the coil, and this electrical current may be communicated along the electrical conduit(s) in navigational guidewire (130) and further to processor (110) via coupling unit (132). This phenomenon may enable IGS navigation system (100) to determine the location of the distal end of navigational guidewire (130) within a three-dimensional space as will be described in greater detail below. In particular, processor (110) executes an algorithm to calculate location coordinates of the distal end of navigational guidewire (130) from the position related signals of the coil(s) in navigational guidewire (130).

In some instances, navigational guidewire (130) is used to generate a three-dimensional model of the anatomy within and adjacent to the patient's nasal cavity; in addition to being used to provide navigation for dilation catheter system (100) within the patient's nasal cavity. Alternatively, any other suitable device may be used to generate a three-dimensional model of the anatomy within and adjacent to the patient's nasal cavity before navigational guidewire (130) is used to provide navigation for dilation catheter system (100) within the patient's nasal cavity. By way of example only, a model of this anatomy may be generated in accordance with at least some of the teachings of U.S. Pub. No. 2016/0310042, entitled "System and Method to Map Structures of Nasal Cavity," published October 27, 2016, the disclosure of which is incorporated by reference herein. Still other suitable ways in which a three-dimensional model of the anatomy within and adjacent to the patient's nasal cavity may be generated will be apparent to those of ordinary skill in the art in view of the teachings herein. It should also be understood that, regardless of how or where the three-dimensional model of the anatomy within and adjacent to the patient's nasal cavity is generated, the model may be stored on console (116). Console (116) may thus render images of at least a portion of the model via display screen (114) and further render real-time video images of the position of navigational guidewire (130) in relation to the model via display screen (114).

### B. Illumination Guidance System

As shown in FIGS. 4-6, an exemplary illuminating guidewire (150) includes a coil (152) positioned about a core wire (154). An illumination fiber (156) extends along the interior of core wire (154) and terminates in an atraumatic lens (158). A connector (155) at a proximal end of illuminating guidewire (150) enables optical coupling between illumination fiber (156) and a light source (not shown). Illumination fiber (156) may comprise one or more optical fibers. Lens (158) is configured to project light when illumination fiber (156) is illuminated by the light source, such that illumination fiber (156) transmits light from the light source to the lens (158). In some versions, a distal end of illuminating guidewire (150) is more flexible than the proximal end of illuminating guidewire (150). Illuminating guidewire (150) has a length enabling the distal end of illuminating guidewire (150) to be positioned distal to balloon (44) *(see* FIG. 1D) while the proximal end of illuminating guidewire (150) is positioned proximal to handle body (22) (*see* FIG. 1D). Illuminating guidewire (150) may include indicia along at least part of its length (e.g., the proximal portion) to provide the operator with visual feedback indicating the depth of insertion of illuminating guidewire (150) relative to dilation catheter (40) (*see* 1D). By way of example only, illuminating guidewire (150) may be configured in accordance with at least some of the teachings of U.S. Pub. No. 2012/0078118, the disclosure of which is incorporated by reference herein. In some versions, illuminating guidewire (150) is configured similar to the Relieva Luma Sentry™ Sinus Illumination System by Acclarent, Inc. of Irvine, California. Other suitable forms that illuminating guidewire (150) may take will be apparent to those of ordinary skill in the art in view of the teachings herein.

### III. Exemplary Guidewire with Core Wire Sleeve

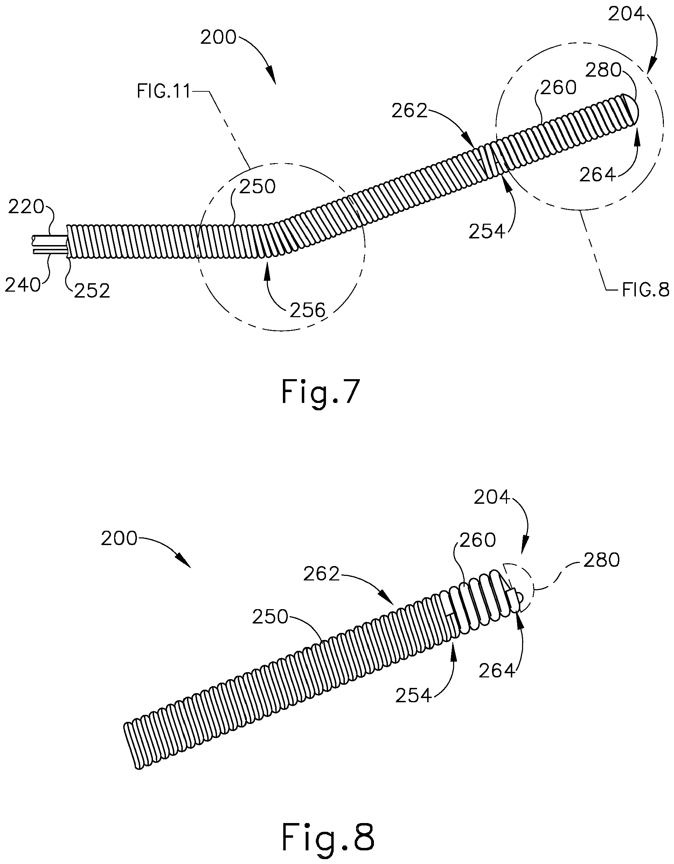

FIG. 7 shows an exemplary alternative guidewire (200) that may be incorporated into dilation instrument assembly (10), in place of guidewire (30, 130, 150). In some versions, at least a portion of the length of guidewire (200) (e.g., approximately 7 inches) is coated in one or more materials. By way of example only, at least a portion of the length of guidewire (200) may be coated in silicone. Other suitable materials that may be used as a coating for guidewire (200) will be apparent to those of ordinary skill in the art in view of the teachings herein. Except as otherwise described below, guidewire (200) is configured and operable similar to any one or more of the various guidewires (30, 130, 150) described above. Guidewire (200) may thus be configured to provide IGS navigation system (100) compatibility or illumination guidance system compatibility to dilation instrument assembly (10).

Guidewire (200) of the present example has a proximal end (not shown), a distal end (204), and an intermediate region (not shown) extending therebetween. Proximal end (not shown) and intermediate region (not shown) are generally constructed as discussed above in other examples provided herein. As best seen in FIGS. 7-9, guidewire (200) includes a proximal coil (250) of helical wire, a distal coil (260) of helical wire, and a core wire assembly (238). A proximal end (252) of proximal coil (250) proximally terminates in a solder joint (not shown), which joins a tubular member (not shown) with proximal coil (250). Proximal coil (250) helically extends from solder joint (not shown) to a distal end (254) of proximal coil and engages with distal coil (260). In the present example, a proximal end (262) of distal coil (260) is joined with distal end (254) of proximal coil (250). Proximal coil (250) includes a preformed bend (256) formed between ends (252, 254). Bend (256) may be bent at an angle in accordance with bend angles known in the art of guidewires that are used in ENT surgical procedures.

In addition, a tip member (280) is secured to a distal end (264) of distal coil (260). Tip member (280) has an atraumatic, dome shape in the present example. In some versions, tip member (280) is formed by adhesive. In some other versions, tip member (280) is formed as a separate piece (e.g., of a polymer) and is then secured to distal end (264), secured to adhesive, or secured to a sensor (not shown), which may be connected to a sensor wire (220). Other suitable ways in which tip member (280) may be formed and secured will be apparent to those of ordinary skill in the art in view of the teachings herein.

With respect to FIGS. 8-10, ends (254, 262) are joined together in an interlocking fashion, such that the overlapping regions of coils (250, 260) form a double helix. More particularly, coils (250, 260) coaxially align along a longitudinal coil axis, which may be straight or bent as discussed above. By way of example only, the interlocking regions of ends (254, 262) may extend along approximately one to two full coil wraps of coils (250, 260). By way of further example only, the interlocking regions of ends (254, 262) may extend along a length between approximately 0.5 mm and approximately 0.75 mm. In the present example, proximal and distal coils (250, 260) are formed of metallic wires (e.g., stainless steel) wrapped in a helical configuration. Also in the present example, a ring of solder (not shown) is applied to the interlocking regions of coils (250, 260) to further secure the interlocking regions of coils (250, 260) together. By way of example only, ring of solder (not shown) may be formed of tin-silver solder. Alternatively, any other suitable material(s) may be used.

In the present example, coils (254, 262) have the same outer diameter but different inner diameters. By way of example only, coils (250, 260) may both have an outer diameter of approximately 0.0345 inches, with proximal coil (250) having an inner diameter of approximately 0.0225 inches, and with distal coil (260) having an inner diameter of approximately 0.0265 inches. Alternatively, any other suitable diameters may be used. Also in the present example, proximal coil (250) has a length of approximately 4.5 inches; while distal coil (260) has a length of approximately 4.25 mm. Alternatively, coils (250, 260) may have any other suitable lengths. Also in the present example, proximal coil (250) has an open pitch of approximately 0.75 mm, in which the open pitch of distal coil (260) is interlocked with a corresponding open pitch, though any other suitable pitch may be used. By way of further example only, the above-noted features of guidewire (200) may be constructed an operable in accordance with at least some of the teachings of U.S. Pat. App. No. 62/453,220, entitled "Navigation Guidewire with Interlocked Coils," filed February 1, 2017, the disclosure of which is incorporated by reference herein.

Core wire assembly (238) is attached within proximal and distal coils (250, 260) and is configured to inhibit longitudinal elongation of the proximal and distal coils (250, 260) along the longitudinal coil axis. To this end, core wire assembly (238) has a core wire (240), a sleeve (270), and a sleeve securement (272). Core wire (240) is proximally secured to tubular member (not shown) within guidewire (200) and extends distally therefrom through proximal and distal coils (250, 260). Core wire (240) is formed of a non-extensible material that provides strength to the region of guidewire (200) along which core wire (240) extends. In particular, core wire (240) inhibits guidewire (200) from stretching longitudinally along the longitudinal coil axis. While core wire (240) is non-extensible in this example, core wire (240) and sleeve (270) are flexible. Moreover, other than the proximal and distal ends of core wire assembly (238), the intermediate region of core wire assembly (238) is not fixedly secured within guidewire (200). Thus, core wire assembly (240) does not adversely affect the lateral flexibility of guidewire (200). By way of example only, the proximal end of core wire (240) may be secured to the inner wall of the tubular member (or some other feature of guidewire (200)) via an adhesive, via an epoxy, or using any other suitable means or techniques as will be apparent to those of ordinary skill in the art in view of the teachings herein.

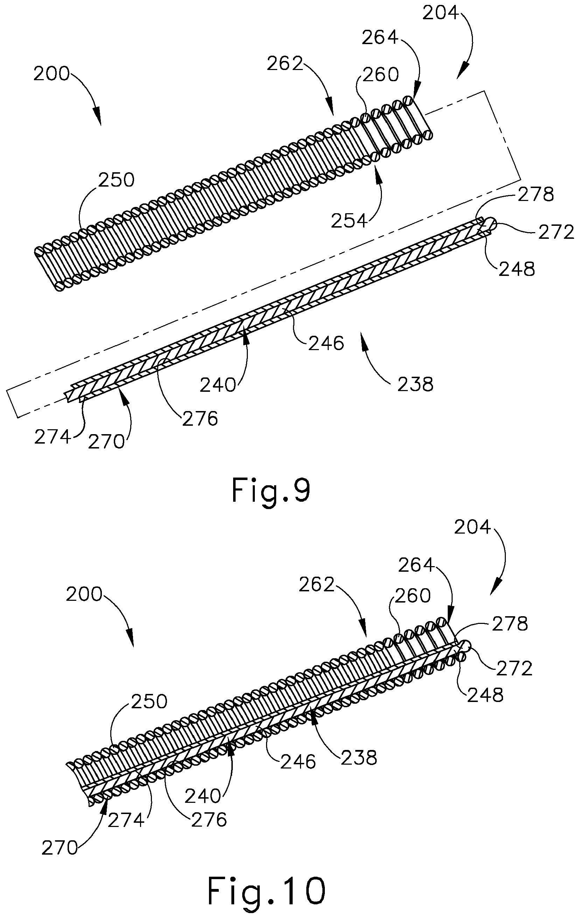

Rather than affix a distal end (248) of core wire (240) directly to distal coil (260), distal end (248) of core wire (240) is secured within sleeve (270), which, in turn, is attached to distal coil (260). In the present example shown in FIGS. 9-10, sleeve (270) is a flexible tube (274) defining a longitudinally extending bore (276) formed from a material configured to be attached to distal coil (260), such as by welding, soldering, or brazing. Core wire (240) is thus selected from available materials to be non-extensible without regard for attachability to distal coil (260), whereas sleeve (270) is selected from available materials for attachability to distal coil (260) without regard for non-extensibility. Sleeve (270) is thus formed from a material different than the material that forms core wire (240).

By way of example only, sleeve (270) may be formed from stainless steel, a nickel titanium alloy, tantalum, niobium, or other metallic material capable of being welded, soldered, or brazed. By way of further example only, core wire (240) may formed from nitinol, nitinol-cobalt, or other material that is non-extensible. While the present example of sleeve (270) is flexible tube (274), it will be appreciated that alternative structures may surround core wire (240), in whole or in part, to form sleeve (270). For example, an alternative sleeve may be formed from wire wrapped about core wire (240) to define an alternative bore. Sleeve (270) is thus not intended to be unnecessarily limited to flexible tube (274). Moreover, in some variations, sleeve (270) is formed from a rigid material.

Core wire (240) is secured within bore (276) of sleeve (270) by longitudinally capturing sleeve (270) on core wire (240) in distal and proximal directions. With respect to distal capture of sleeve (270), sleeve securement (272) is a bulbous sleeve securement (272) attached to a distal tip (278) of core wire (240). In some versions, bulbous sleeve securement (272) is formed by pressing the distal end of core wire (240) or otherwise forming core wire (240) to define bulbous sleeve securement (272). Bulbous sleeve securement (272) defines an outer bulbous diameter, which is larger than an inner diameter of bore (276) through sleeve (270). Sleeve (270) thus abuts against bulbous sleeve securement (272) such that bulbous sleeve securement (272) inhibits distal movement of sleeve (270) relative to core wire (240). Bulbous sleeve securement (272) extends distally beyond distal end (264) of distal coil (260) and radially within the outer diameter of distal coil (260). However, it will be appreciated that bulbous sleeve securement (272) may be alternatively positioned relative to distal coil (260). In an alternative sleeve securement, distal tip (278) may be attached, such as by being bonded, to tip member (280) (*see* FIG. 8) to inhibit distal movement of sleeve (270) relative to core wire (240). The invention is thus not intended to be unnecessarily limited to bulbous sleeve securement (272) shown in the present example.

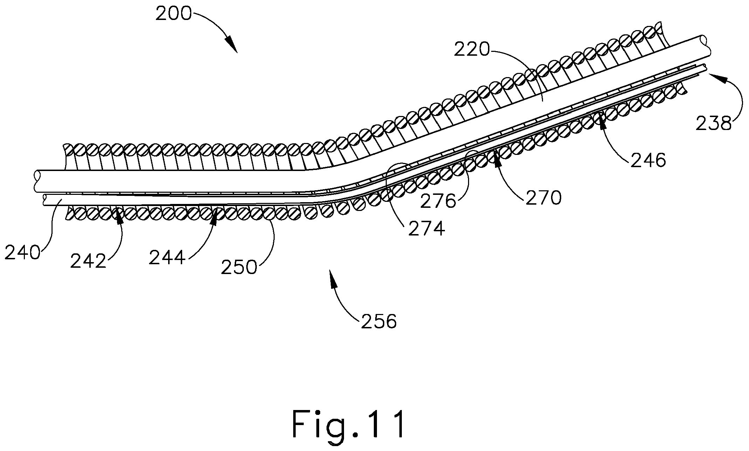

FIG. 11 illustrates proximal capture of sleeve (270) on core wire (240) in greater detail. In the present example, core wire (240) tapers just proximal to bend (256). More particularly, core wire (240) has a proximal region (242) that has an outer diameter that is larger than an outer diameter of a distal region (246), with a tapered region (244) providing a smooth transition between these outer diameters along bend (256). The outer diameter of proximal region (242) is generally larger than the inner diameter of sleeve (270). Sleeve (270) thus abuts against the outer diameter of proximal region (242) such that proximal region (242) of core wire (240) inhibits proximal movement of sleeve (270) relative to core wire (240). Thus, sleeve (270) is longitudinally captured between the outer diameter of proximal region (242) and bulbous sleeve securement (272) (*see* FIG. 10). In some variations, core wire (240) defines a stepped surface, such that the proximal end of sleeve (270) abuts the stepped surface; and such that the stepped surface of core wire (240) prevents proximal movement of sleeve (270) relative to core wire (240). Such a stepped surface may be provided in addition to or in lieu of tapered region (244) in core wire (240). Also in some variations, the outer diameter of sleeve (270) is substantially equal to the outer diameter of proximal region (242).

By way of example only, proximal region (242) may have an outer diameter of approximately 0.0095 inches or approximately 0.10 inches. By way of further example only, distal region (246) may be flattened to a thickness of approximately 0.0027 inches. Various suitable diameters, thicknesses, and taper angles that may be used along tapered region (244) will be apparent to those of ordinary skill in the art in view of the teachings herein. While the core wire (240) and sleeve (270) have one or more tapers for receiving and abutting sleeve (270) against core wire (240), alternative examples of core wire and sleeve may instead have respective steps that abut against each other. The invention is thus not intended to be unnecessarily limited to the tapers described herein for capturing sleeve (270) on core wire (240).

In manufacture, core wire (240) is inserted through bore (276) of sleeve (270) such that sleeve (270) abuts against the outer diameter of proximal region (242). Bulbous sleeve securement (272) is then attached to distal tip (278) of core wire (240), such as by welding, to capture sleeve (270) between the outer diameter of proximal region (242) and bulbous sleeve securement (272). Alternatively, bulbous sleeve securement (272) is formed by pressing distal tip (278) or otherwise forming bulbous sleeve securement (272) as a unitary, integral feature of core wire (240). Core wire assembly (238) may then be inserted through proximal and distal coils (250, 260) to align sleeve (270) against distal coil (260). Sleeve (270) is attached to distal coil (260), such as by welding, soldering, or brazing, to inhibit longitudinal elongation of the proximal and distal wire coils along the longitudinal coil axis. The distal portion of core wire (240) is not directly welded to distal coil (260) in this example.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus, comprising: (a) a helical wire coil; and (b) a core wire assembly including: (i) a core wire extending through the helical wire coil, wherein the core wire is formed from a first material, wherein the first material is non-extensible, and (ii) a sleeve having a longitudinal bore, wherein the core wire is disposed within the longitudinal bore of the sleeve, wherein the core wire is secured within the sleeve, wherein the sleeve is formed from a second material, wherein the second material of the sleeve is different than the first material of the core wire, wherein the sleeve is fixedly secured to a distal portion of the helical wire coil such that core wire assembly inhibits longitudinal elongation of the helical wire coil along a longitudinal coil axis.

### Example 2

The apparatus of Example 1, wherein the core wire has a proximal region with a proximal outer diameter and a distal region with a distal outer diameter, and wherein the proximal outer diameter is larger than the distal outer diameter.

### Example 3

The apparatus of Example 2, wherein the proximal region of the core wire tapers to the distal region of the core wire.

### Example 4

The apparatus of any one or more of Examples 2 through 3, wherein the sleeve abuts against the proximal outer diameter of the proximal region such that the proximal region of the core wire inhibits proximal longitudinal movement of the sleeve relative to the core wire.

### Example 5

The apparatus of any one or more of Examples 1 through 4, wherein the core wire assembly further includes a sleeve securement at a distal end of the core wire, wherein the sleeve securement is configured to engage the sleeve and thereby secure the sleeve relative to the core wire.

### Example 6

The apparatus of Example 5, wherein the sleeve securement abuts against the sleeve and is configured to inhibit distal longitudinal movement of the sleeve relative to the core wire.

### Example 7

The apparatus of Example 6, wherein the longitudinal bore has an inner diameter, wherein the sleeve securement comprises a bulbous sleeve securement having a bulbous outer diameter, wherein the bulbous outer diameter is larger than the inner diameter of the longitudinal bore.

### Example 8

The apparatus of Example 7, wherein the bulbous sleeve securement is attached to a distal tip of the core wire.

### Example 9

The apparatus of Example 8, wherein the bulbous sleeve securement is welded to the distal tip of the core wire.

### Example 10

The apparatus of any one or more of Examples 1 through 9, wherein the first material and the second material are configured to be brazed, soldered, or welded together.

### Example 11

The apparatus of Example 10, wherein the first material and the second material are configured to be welded together.

### Example 12

The apparatus of any one or more of Examples 1 through 11, wherein the first material is selected from the group consisting of nitinol or nitinol-cobalt, and wherein the second material is selected from the group consisting of stainless steel, tantalum, or niobium.

### Example 13

The apparatus of any one or more of Examples 1 through 12, wherein the helical wire coil includes a preformed bend, and wherein the core wire assembly extends through the preformed bend of the helical wire coil.

### Example 14

The apparatus of Example 13, wherein the core wire has a proximal region with a proximal outer diameter and a distal region with a distal outer diameter, and wherein the proximal outer diameter is proximal to the preformed bend.

### Example 15

The apparatus of any one or more of Examples 1 through 14, further comprising:
(a) a body; (b) a guide extending distally from the body; (c) a guidewire including the helical wire coil and the core wire assembly, wherein the guidewire is slidably disposed relative to the guide; and (d) a dilation catheter slidably disposed relative to the guidewire, wherein the dilation catheter includes an expandable dilator.

### Example 16

An apparatus, comprising: (a) a proximal wire coil, wherein the proximal wire coil is helical; (b) a distal wire coil, wherein the distal wire coil is helical and interlocked with the proximal wire coil such that the proximal and distal wire coils form a double helix configuration extending along a longitudinal coil axis; and (c) a core wire assembly including: (i) a core wire extending through the proximal and distal wire coils, wherein the core wire is formed from a first material, wherein the first material is non-extensible and further includes: (A) a proximal region with a proximal outer diameter, and (B) a distal region with a distal outer diameter, and wherein the proximal outer diameter is larger than the distal outer diameter, (ii) a sleeve having a longitudinal bore and receiving the core wire therein, wherein the core wire is secured within the sleeve and formed from a second material, wherein the second material of the sleeve is different than the first material of the core wire, wherein the sleeve abuts against the proximal outer diameter of the proximal region such that the proximal region of the core wire inhibits proximal longitudinal movement of the sleeve relative to the core wire, and (iii) a sleeve securement attached to the core wire, wherein the sleeve securement abuts against the sleeve and is configured to inhibit distal longitudinal movement of the sleeve relative to the core wire such that the sleeve is captured on the core wire between the proximal outer diameter of the core wire and the sleeve securement, wherein the sleeve is attached to the distal wire coil such that core wire inhibits longitudinal elongation of the proximal and distal wire coils along the longitudinal coil axis.

### Example 17

The apparatus of Example 16, wherein the first material and the second material are configured to be brazed, soldered, or welded together.

### Example 18

The apparatus of any one or more of Examples 16 through 17, wherein the proximal wire coil includes a preformed bend, wherein the preformed bend is positioned proximal to a distal end of the proximal wire coil, and wherein the core wire assembly extends through the preformed bend of the proximal wire coil.

### Example 19

The apparatus of any one or more of Examples 16 through 18, further comprising:
(a) a body; (b) a guide extending distally from the body; (c) a guidewire including the proximal wire coil, the distal wire coil, and the core wire assembly, wherein the guidewire is slidably disposed relative to the guide; and (d) a dilation catheter slidably disposed relative to the guidewire, wherein the dilation catheter includes an expandable dilator.

### Example 20

A method of manufacturing a guidewire for a dilation catheter, the method comprising: (a) inserting a core wire formed of a first metallic material through a longitudinal bore in a sleeve formed of a second metallic material, wherein the core wire is non-extensible; (b) securing the core wire within the sleeve; and (c) attaching a distal portion of the sleeve to a helical wire coil within an interior defined by the helical wire coil, wherein the core wire and the sleeve prevent elongation of the helical wire coil, wherein the core wire and the sleeve permit lateral deformation of the helical wire coil.

### V. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a helical wire coil; and
(b) a core wire assembly including:
(i) a core wire extending through the helical wire coil, wherein the core wire is formed from a first material, wherein the first material is non-extensible, and
(ii) a sleeve having a longitudinal bore, wherein the core wire is disposed within the longitudinal bore of the sleeve, wherein the core wire is secured within the sleeve, wherein the sleeve is formed from a second material, wherein the second material of the sleeve is different than the first material of the core wire,
wherein the sleeve is fixedly secured to a distal portion of the helical wire coil such that core wire assembly inhibits longitudinal elongation of the helical wire coil along a longitudinal coil axis.

2. The apparatus of claim 1, wherein the core wire has a proximal region with a proximal outer diameter and a distal region with a distal outer diameter, and wherein the proximal outer diameter is larger than the distal outer diameter.

3. The apparatus of claim 2, wherein the proximal region of the core wire tapers to the distal region of the core wire, or wherein the sleeve abuts against the proximal outer diameter of the proximal region such that the proximal region of the core wire inhibits proximal longitudinal movement of the sleeve relative to the core wire.

4. The apparatus of claim 1, wherein the core wire assembly further includes a sleeve securement at a distal end of the core wire, wherein the sleeve securement is configured to engage the sleeve and thereby secure the sleeve relative to the core wire.

5. The apparatus of claim 4, wherein the sleeve securement abuts against the sleeve and is configured to inhibit distal longitudinal movement of the sleeve relative to the core wire.

6. The apparatus of claim 5, wherein the longitudinal bore has an inner diameter, wherein the sleeve securement comprises a bulbous sleeve securement having a bulbous outer diameter, wherein the bulbous outer diameter is larger than the inner diameter of the longitudinal bore.

7. The apparatus of claim 6, wherein the bulbous sleeve securement is attached to a distal tip of the core wire, preferably wherein the bulbous sleeve securement is welded to the distal tip of the core wire.

8. The apparatus of claim 1, wherein the helical wire coil includes a preformed bend, and wherein the core wire assembly extends through the preformed bend of the helical wire coil, preferably wherein the core wire has a proximal region with a proximal outer diameter and a distal region with a distal outer diameter, and wherein the proximal outer diameter is proximal to the preformed bend.

9. The apparatus of claim 1, further comprising:
(a) a body;
(b) a guide extending distally from the body;
(c) a guidewire including the helical wire coil and the core wire assembly, wherein the guidewire is slidably disposed relative to the guide; and
(d) a dilation catheter slidably disposed relative to the guidewire, wherein the dilation catheter includes an expandable dilator.

10. An apparatus, comprising:
(a) a proximal wire coil, wherein the proximal wire coil is helical;
(b) a distal wire coil, wherein the distal wire coil is helical and interlocked with the proximal wire coil such that the proximal and distal wire coils form a double helix configuration extending along a longitudinal coil axis; and
(c) a core wire assembly including:
(i) a core wire extending through the proximal and distal wire coils, wherein the core wire is formed from a first material, wherein the first material is non-extensible and further includes:
(A) a proximal region with a proximal outer diameter, and
(B) a distal region with a distal outer diameter, and wherein the proximal outer diameter is larger than the distal outer diameter,
(ii) a sleeve having a longitudinal bore and receiving the core wire therein, wherein the core wire is secured within the sleeve and formed from a second material, wherein the second material of the sleeve is different than the first material of the core wire, wherein the sleeve abuts against the proximal outer diameter of the proximal region such that the proximal region of the core wire inhibits proximal longitudinal movement of the sleeve relative to the core wire, and
(iii) a sleeve securement attached to the core wire, wherein the sleeve securement abuts against the sleeve and is configured to inhibit distal longitudinal movement of the sleeve relative to the core wire such that the sleeve is captured on the core wire between the proximal outer diameter of the core wire and the sleeve securement,
wherein the sleeve is attached to the distal wire coil such that core wire inhibits longitudinal elongation of the proximal and distal wire coils along the longitudinal coil axis.

11. The apparatus of claim 1 or 10, wherein the first material and the second material are configured to be brazed, soldered, or welded together.

12. The apparatus of claim 11 when dependent upon claim 1, wherein the first material and the second material are configured to be welded together, or wherein the first material is selected from the group consisting of nitinol or nitinol-cobalt, and wherein the second material is selected from the group consisting of stainless steel, tantalum, or niobium.

13. The apparatus of claim 10, wherein the proximal wire coil includes a preformed bend, wherein the preformed bend is positioned proximal to a distal end of the proximal wire coil, and wherein the core wire assembly extends through the preformed bend of the proximal wire coil.

14. The apparatus of claim 10, further comprising:
(a) a body;
(b) a guide extending distally from the body;
(c) a guidewire including the proximal wire coil, the distal wire coil, and the core wire assembly, wherein the guidewire is slidably disposed relative to the guide; and
(d) a dilation catheter slidably disposed relative to the guidewire, wherein the dilation catheter includes an expandable dilator.

15. A method of manufacturing a guidewire for a dilation catheter, the method comprising:
(a) inserting a core wire formed of a first metallic material through a longitudinal bore in a sleeve formed of a second metallic material, wherein the core wire is non-extensible;
(b) securing the core wire within the sleeve; and
(c) attaching a distal portion of the sleeve to a helical wire coil within an interior defined by the helical wire coil, wherein the core wire and the sleeve prevent elongation of the helical wire coil, wherein the core wire and the sleeve permit lateral deformation of the helical wire coil.
